# EUROPEAN PATENT SPECIFICATION

(11) **EP 2 817 064 B1**
(45) Date of publication and mention of the grant of the patent: **15.11.2017**
(21) Application number: 13708315.0
(22) Date of filing: 22.02.2013
(51) Int. Cl.: A61N 1/08, A61N 1/375

(54) **SYSTEMS FOR MODIFYING IMPEDANCE ALONG ELECTRICAL PATHS OF ELECTRICAL STIMULATION SYSTEMS**
SYSTEME ZUR ÄNDERUNG DER IMPEDANZ ENTLANG ELEKTRISCHER PFADE VOM ELEKTRISCHEN STIMULATIONSSYSTEMEN
SYSTÈMES POUR MODIFIER L'IMPÉDANCE LE LONG DES PASSAGES ÉLECTRIQUES DE SYSTÈMES DE STIMULATION ÉLECTRIQUE

(30) Priority: 24.02.2012 US 201261602881 P
(43) Date of publication of application: 31.12.2014
(73) Proprietor: Boston Scientific Neuromodulation Corporation, Valencia, CA 91355 (US)
(72) Inventor: MURTONEN, Salomo, Pasadena, CA 91107 (US); CARBUNARU, Rafael, Valley Village, CA 91607 (US); BOCEK, Joseph, M., Seattle, WA 98102 (US); GUPTA, Gaurav, Valencia, CA 91354 (US)
(74) Representative: Vossius & Partner Patentanwälte Rechtsanwälte mbB
(86) International application number: PCT/US2013/027298
(87) International publication number: WO 2013/126682

(56) References cited:
- EP-A1- 2 322 242
- WO-A1-2008/100319
- WO-A1-2012/112490
- WO-A2-2009/117599
- US-A1- 2011 029 036

## Description

### FIELD

The present description is directed to the area of implantable electrical stimulation systems and methods of making and using the systems. The present description is also directed to impedance circuitries for modifying impedances of electrical paths of systems during exposure of patients to applied electromagnetic fields, as well as methods of making and using the impedance circuitries and electrical stimulation systems. The invention is set out in the appended claims.

### BACKGROUND

Implantable electrical stimulation systems have proven therapeutic in a variety of diseases and disorders. For example, spinal cord stimulation systems have been used as a therapeutic modality for the treatment of chronic pain syndromes. Peripheral nerve stimulation has been used to treat chronic pain syndrome and incontinence, with a number of other applications under investigation. Functional electrical stimulation systems have been applied to restore some functionality to paralyzed extremities in spinal cord injury patients.

Stimulators have been developed to provide therapy for a variety of treatments. A stimulator can include a control module (with a pulse generator), one or more leads, and an array of stimulator electrodes on each lead. The stimulator electrodes are in contact with or near the nerves, muscles, or other tissue to be stimulated. The pulse generator in the control module generates electrical pulses that are delivered by the electrodes to body tissue.

Conventional implanted electrical stimulation systems are often incompatible with magnetic resonance imaging ("MRI") due to the large radio frequency ("RF") pulses used during MRI. The RF pulses can generate transient signals in the conductors and electrodes of an implanted lead. These signals can have deleterious effects
including, for example, unwanted heating of the tissue causing tissue damage, induced currents in the lead, or premature failure of electronic EP-A-2 322 242 discloses a filtering scheme for an implantanble medical device that mitigates potentially adverse effects that may be caused by MRI-induced signals. WO-A-2009/117599 discloses a shielded three-terminal flat-through EMI/energy dissipating filter for an implantable medical device.

### BRIEF SUMMARY

In one embodiment, an implantable medical device system for providing electrical stimulation includes a control module configured and arranged to electrically couple to a lead. The control module includes a sealed housing having an interior and an exterior, an electronic subassembly disposed in the interior of the housing, and a connector assembly coupled to the exterior of the housing. The connector assembly defines a port configured and arranged for receiving the lead. A plurality of connector contacts are disposed in the port. The connector contacts are configured and arranged to electrically couple with terminals of the lead when the lead is operationally received by the port. The plurality of connector contacts include a first connector contact and a second connector contact. A plurality of feedthrough interconnects extend from the connector assembly into the interior of the sealed housing. The plurality of feedthrough interconnects electrically couple the plurality of connector contacts to the electronic subassembly. The plurality of feedthrough interconnects include a first feedthrough interconnect and a second feedthrough interconnect. The first feedthrough interconnect electrically couples the first connector contact to the electronic subassembly and the second feedthrough interconnect electrically couples the second connector contact to the electronic subassembly. Impedance circuitry is disposed in the control module and is configured and arranged for modulating impedance associated with terminals and conductors of the lead. The impedance circuitry includes a plurality of impedance elements. Each of the plurality of impedance elements is electrically coupled to a different feedthrough interconnect of the plurality of feedthrough interconnects. Each impedance element of the plurality of impedance elements has a pre-defined impedance. The plurality of impedance elements include a first impedance element electrically coupled to the first feedthrough interconnect and a second impedance element electrically coupled to the second feedthrough interconnect. The pre-defined impedance of the first impedance element is different than the pre-defined impedance of the second impedance element.

In another embodiment, an implantable medical device system for providing electrical stimulation includes a control module configured and arranged to electrically couple to a lead. The control module includes a sealed housing having an interior and an exterior, an electronic subassembly disposed in the interior of the housing, and a connector assembly coupled to the exterior of the housing. The connector assembly defines a port configured and arranged for receiving the lead. A plurality of connector contacts are disposed in the port. The connector contacts are configured and arranged to electrically couple with terminals of the lead when the lead is operationally received by the port. A plurality of feedthrough interconnects extend from the connector assembly into the interior of the sealed housing. The plurality of feedthrough interconnects electrically couple the plurality of connector contacts to the electronic subassembly. Impedance circuitry is disposed in the control module and is configured and arranged for modulating impedance associated with terminals and conductors of the lead. The impedance circuitry includes a plurality of impedance elements. Each of the plurality of impedance elements is electrically coupled to a different feedthrough interconnect of the plurality of feedthrough interconnects. At least one of the impedance elements is configured and arranged for enabling adjustment of impedance along the feedthrough interconnect to which the at least one of the impedance elements is electrically coupled.

In yet another embodiment, an implantable medical device system for providing electrical stimulation includes a control module configured and arranged to electrically couple to a lead. The control module includes a sealed housing having an interior and an exterior, an electronic subassembly disposed in the interior of the housing, and a connector assembly coupled to the exterior of the housing. The connector assembly defines a port. A plurality of connector contacts are disposed in the port. The connector contacts are configured and arranged to electrically couple with terminals of the lead when the lead is operationally received by the port. The plurality of connector contacts includes a first connector contact and a second connector contact. A plurality of feedthrough interconnects extend from the connector assembly into the interior of the sealed housing. The plurality of feedthrough interconnects electrically couple the plurality of connector contacts to the electronic subassembly. The plurality of feedthrough interconnects include a first feedthrough interconnect and a second feedthrough interconnect. The first feedthrough interconnect electrically couples the first connector contact to the electronic subassembly and the second feedthrough interconnect
electrically couples the second connector contact to the electronic subassembly. An adapter is electrically coupleable to the connector assembly. The adapter includes impedance circuitry configured and arranged for modulating impedance associated with terminals and conductors of the lead. The impedance circuitry includes a plurality of impedance elements. Each of the plurality of impedance elements is electrically coupleable to a different connector contact of the plurality of connector contacts. The plurality of impedance elements either have impedances that are pre-defmed or include at least one impedance element that is configured and arranged for enabling adjustment of impedance along the feedthrough interconnect to which the at least one impedance element is electrically coupled. When the plurality of impedance elements have impedances that are pre-defined, the plurality of impedance elements include a first impedance element electrically coupled to the first feedthrough interconnect and a second impedance element electrically coupled to the second feedthrough interconnect. The pre-defined impedance of the first impedance element is different than the pre-defined impedance of the second impedance element.

The present invention is set out in the appended claims. The embodiments, aspects or examples according to the present description that do not fall within the scope of said claims are provided for illustrative purposes only and do not form part of the present invention.

### BRIEF DESCRIPTION OF THE DRAWINGS

Non-limiting and non-exhaustive embodiments of the present description are described with reference to the following drawings. In the drawings, like reference numerals refer to like parts throughout the various figures unless otherwise specified.

For a better understanding of the present invention, reference will be made to the following Detailed Description, which is to be read in association with the accompanying drawings, wherein:
FIG. 1 is a schematic view of one embodiment of an electrical stimulation system that includes a paddle lead with a paddle body coupled to a control module via lead bodies, according to the description;
FIG. 2 is a schematic view of another embodiment of an electrical stimulation system that includes a percutaneous lead coupled to the control module of FIG. 1, according to the description;
FIG. 3A is a schematic view of one embodiment of a connector assembly disposed in the control module of FIG. 1, the connector assembly configured and arranged to receive the proximal portion of one of the lead bodies of FIG. 1, according to the description;
FIG. 3B is a schematic view of one embodiment of a plurality of connector assemblies disposed in the control module of FIG. 1, the connector assemblies configured and arranged to receive the proximal portions of the lead bodies of FIG. 1, according to the description;
FIG. 3C is a schematic view of one embodiment of a proximal portion of one of the lead bodies of FIG. 1, a lead extension, and the control module of FIG. 1, the lead extension configured and arranged to couple the lead body to the control module, according to the description;
FIG. 4 is a schematic view of one embodiment of a portion of a lead inserted into a connector assembly of a control module having a sealed housing, the connector assembly having a plurality of connector contacts that are electrically coupled to an electronic subassembly in the sealed housing via a plurality of feedthrough interconnects, according to the description;
FIG. 5A is a schematic view of one embodiment of impedance circuitry disposed between the connector contacts of FIG. 4 and the electronic subassembly of FIG. 4, according to the description;
FIG. 5B is a schematic view of another embodiment of the impedance circuitry of FIG. 5A disposed between the connector contacts of FIG. 4 and the electronic subassembly of FIG. 4, according to the description;
FIG. 5C is a schematic view of yet another embodiment of the impedance circuitry of FIG. 5A disposed between the connector contacts of FIG. 4 and the electronic subassembly of FIG. 4, according to the description;
FIG. 6 is a schematic view of another embodiment of the impedance circuitry of FIG. 5A, the impedance circuitry disposed between terminals of the lead of FIG. 4 and the connector contacts of FIG. 4, according to the description; and
FIG. 7 is a schematic overview of one embodiment of components of a stimulation system, including an electronic subassembly disposed within a control module, according to the description.

### DETAILED DESCRIPTION

The present description is directed to the area of implantable electrical stimulation systems and methods of making and using the systems. The present description is also directed to impedance circuitries for modifying impedances of electrical paths of systems during exposure of patients to applied electromagnetic fields, as well as methods of making and using the impedance circuitries and electrical stimulation systems. The invention is set out in the appended claims.

Suitable implantable electrical stimulation systems include, but are not limited to, an electrode lead ("lead") with one or more electrodes disposed on a distal end of the lead and one or more terminals disposed on one or more proximal ends of the lead. Leads include, for example, percutaneous leads, paddle leads, and cuff leads. Examples of electrical stimulation systems with leads are found in, for example, U.S. Patents Nos. 6,181,969; 6,516,227; 6,609,029; 6,609,032; 6,741,892; 7,244,150; 7,672,734; 7,761,165; 7,949,395; 7,974,706; 8,175,710; and 8,224,450; and U.S. Patent Applications Publication Nos. 2005/0165465 and 2007/0150036.

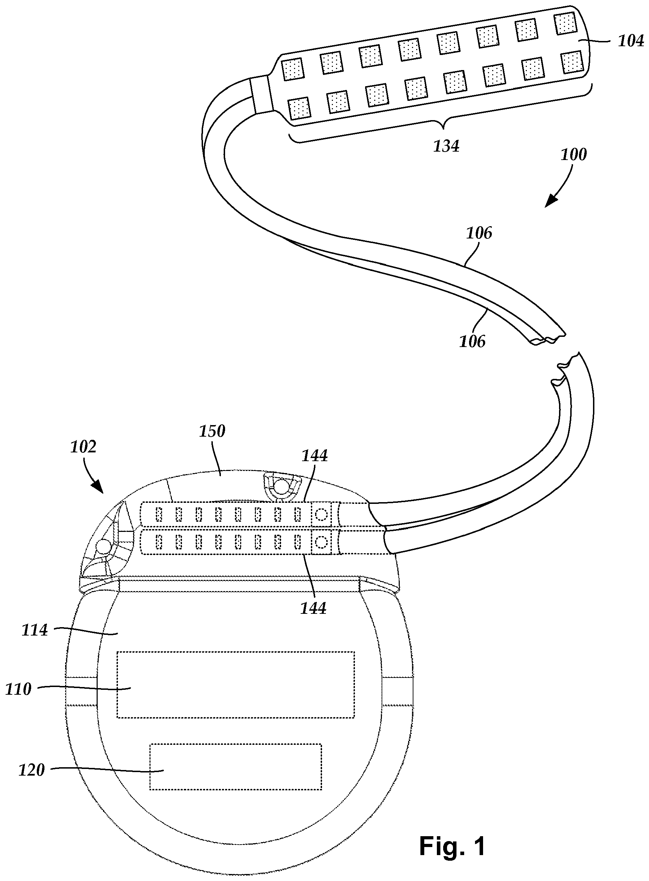

Figure 1 illustrates schematically one embodiment of an electrical stimulation system 100. The electrical stimulation system includes a control module (*e.g.,* a stimulator or pulse generator) 102, a paddle body 104, and one or more lead bodies 106 coupling the control module 102 to the paddle body 104. The paddle body 104 and the one or more lead bodies 106 form a lead. The paddle body 104 typically includes an array of electrodes 134. The control module 102 typically includes an electronic subassembly 110 and an optional power source 120 disposed in a sealed housing 114. In Figure 1, two lead bodies 106 are shown coupled to the control module 102.

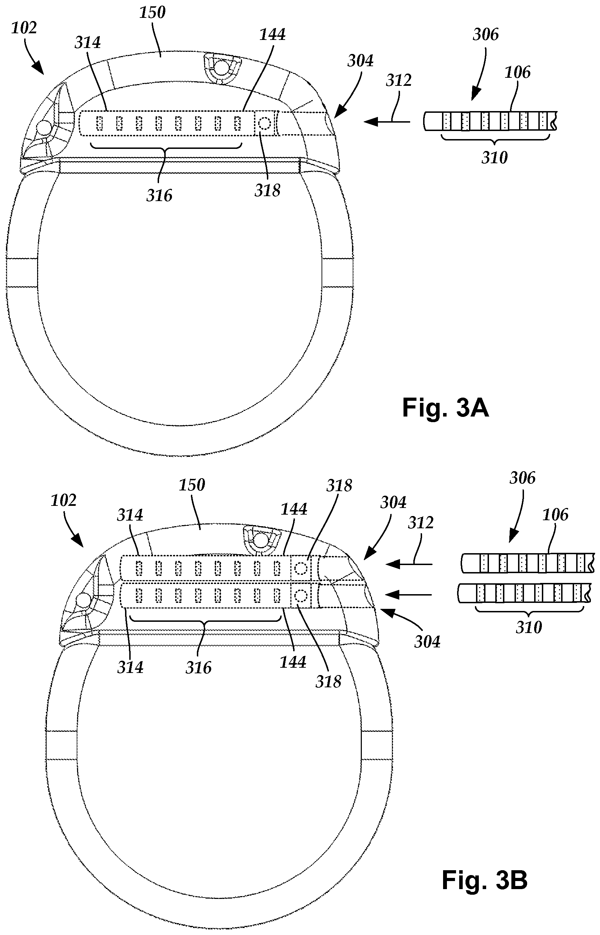

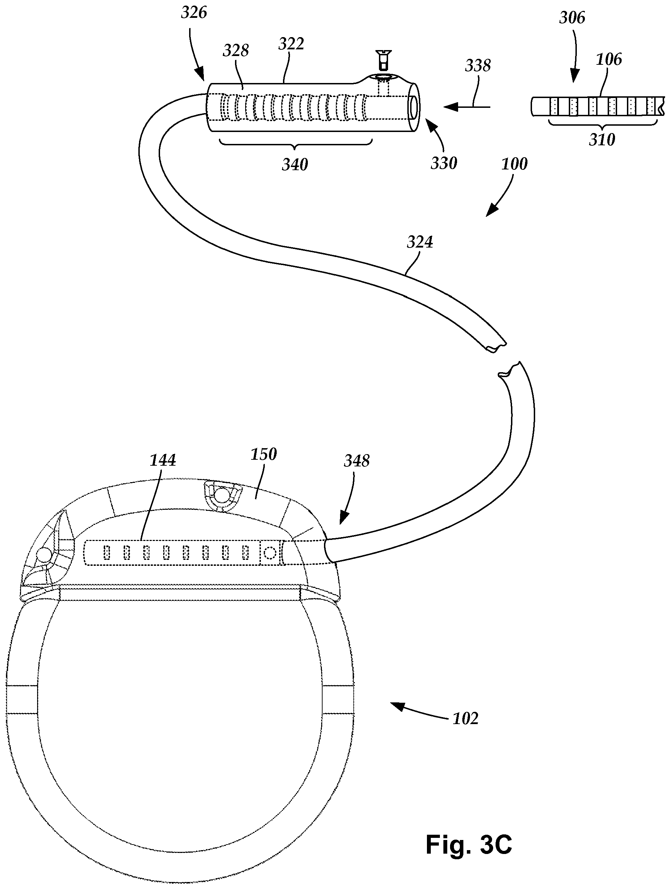

The control module 102 typically includes one or more connector assemblies 144 into which the proximal end of the one or more lead bodies 106 can be plugged to make an electrical connection via connector contacts (*e.g.,* 316 in Figures 3A-3B; and 340 of Figure 3C) disposed in the connector assembly 144 and terminals (*e.g*., 310 in Figures 3A-3C) on each of the one or more lead bodies 106. The connector contacts are coupled to the electronic subassembly 110 and the terminals are coupled to the electrodes 134. In Figure 1, two connector assemblies 144 are shown.

The one or more connector assemblies 144 may be disposed in a header 150. The header 150 provides a protective covering over the one or more connector assemblies 144. The header 150 may be formed using any suitable process including, for example, casting, molding (including injection molding), and the like. In addition, one or more lead extensions 324 (see Figure 3C) can be disposed between the one or more lead bodies 106 and the control module 102 to extend the distance between the one or more lead bodies 106 and the control module 102.

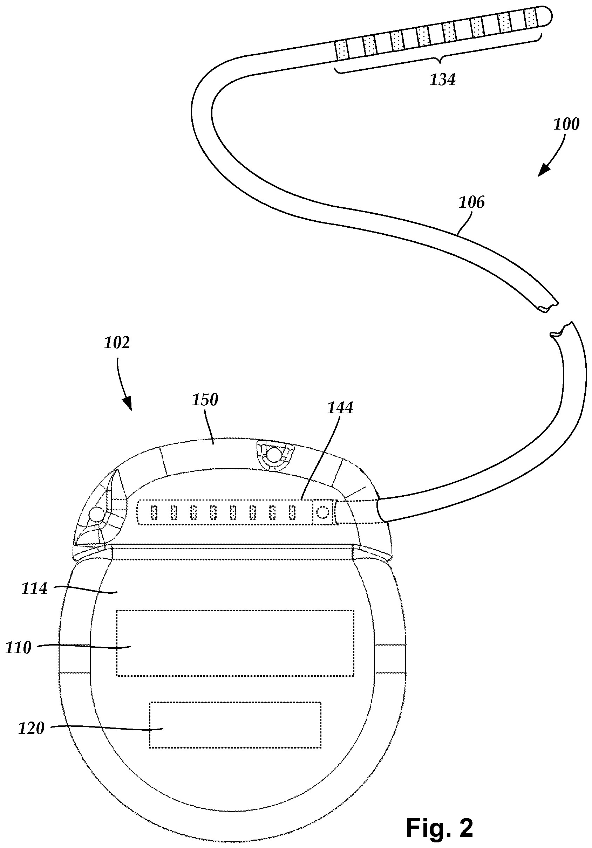

It will be understood that the electrical stimulation system can include more, fewer, or different components and can have a variety of different configurations including those configurations disclosed in the electrical stimulation system references cited herein. For example, instead of a paddle body 104, the electrodes 134 can be disposed in an array at or near the distal end of the lead body 106 forming a percutaneous lead, as illustrated in Figure 2. A percutaneous lead may be isodiametric along the length of the lead body 106.

The electrical stimulation system or components of the electrical stimulation system, including one or more of the lead bodies 106, the control module 102, and, in the case of a paddle lead, the paddle body 104, are typically implanted into the body of a patient. The electrical stimulation system can be used for a variety of applications including, but not limited to, spinal cord stimulation, brain stimulation, neural stimulation, muscle stimulation, cardiac stimulation, and the like.

The electrodes 134 can be formed using any conductive, biocompatible material. Examples of suitable materials include metals, alloys, conductive polymers, conductive carbon, and the like, as well as combinations thereof. In at least some embodiments, one or more of the electrodes 134 are formed from one or more of: platinum, platinum iridium, palladium, titanium, or rhenium.

The number of electrodes 134 in the array of electrodes 134 may vary. For example, there can be two, three, four, five, six, seven, eight, nine, ten, eleven, twelve, thirteen, fourteen, fifteen, sixteen, or more electrodes 134. As will be recognized, other numbers of electrodes 134 may also be used. As will be recognized, other numbers of electrodes 134 may also be used. In Figure 1, sixteen electrodes 134 are shown. The electrodes 134 can be formed in any suitable shape including, for example, round, oval, triangular, rectangular, pentagonal, hexagonal, heptagonal, octagonal, or the like.

The electrodes of the paddle body 104 or one or more lead bodies 106 are typically disposed in, or separated by, a non-conductive, biocompatible material including, for example, silicone, polyurethane, and the like or combinations thereof. The paddle body 104 and one or more lead bodies 106 may be formed in the desired shape by any process including, for example, molding (including injection molding), casting, and the like. Electrodes and connecting wires can be disposed onto or within a paddle body either prior to or subsequent to a molding or casting process. The non-conductive material typically extends from the distal end of the lead to the proximal end of each of the one or more lead bodies 106. The non-conductive, biocompatible material of the paddle body 104 and the one or more lead bodies 106 may be the same or different. The paddle body 104 and the one or more lead bodies 106 may be a unitary structure or can be formed as two separate structures that are permanently or detachably coupled together.

Terminals (*e.g*., 310 in Figures 3A-3C) are typically disposed at the proximal end of the one or more lead bodies 106 for connection to corresponding conductive contacts (*e.g.,* 316 in Figures 3A-3B; and 340 of Figure 3C) in connector assemblies (*e.g.,* 144 in Figures 1-3C) disposed on, for example, the control module 102 (or to other devices, such as conductive contacts on a lead extension, an operating room cable, a splitter, an adaptor, or the like).

Conductive wires (not shown) extend from the terminals (*e.g.,* 310 in Figures 3A-3C) to the electrodes 134. Typically, one or more electrodes 134 are electrically coupled to a terminal (*e.g.,* 310 in Figures 3A-3C). In some embodiments, each terminal (*e.g.,* 310 in Figures 3A-3C) is only coupled to one electrode 134.

The conductive wires may be embedded in the non-conductive material of the lead or can be disposed in one or more lumens (not shown) extending along the lead. In some embodiments, there is an individual lumen for each conductive wire. In other embodiments, two or more conductive wires may extend through a lumen. There may also be one or more lumens (not shown) that open at, or near, the proximal end of the lead, for example, for inserting a stylet rod to facilitate placement of the lead within a body of a patient. Additionally, there may also be one or more lumens (not shown) that open at, or near, the distal end of the lead, for example, for infusion of drugs or medication into the site of implantation of the paddle body 104. In at least one embodiment, the one or more lumens may be flushed continually, or on a regular basis, with saline, epidural fluid, or the like. In at least some embodiments, the one or more lumens can be permanently or removably sealable at the distal end.

As discussed above, the one or more lead bodies 106 may be coupled to the one or more connector assemblies 144 disposed on the control module 102. The control module 102 can include any suitable number of connector assemblies 144 including, for example, two three, four, five, six, seven, eight, or more connector assemblies 144. It will be understood that other numbers of connector assemblies 144 may be used instead. In Figure 1, each of the two lead bodies 106 includes eight terminals that are shown coupled with eight conductive contacts disposed in a different one of two different connector assemblies 144.

In at least some embodiments, leads are coupled to connectors disposed on control modules. Figure 3A is a schematic perspective view of one embodiment of a single connector assembly 144 disposed on the control module 102. Figure 3B is a schematic perspective view of one embodiment of a plurality of connector assemblies 144 disposed on the control module 102. In at least some embodiments, the control module 102 includes two connector assemblies 144. In at least some embodiments, the control module 102 includes four connector assemblies 144.

In Figures 3A and 3B, the proximal ends 306 of one or more lead bodies 106 are shown configured and arranged for insertion to the control module 102. In Figures 3A and 3B, the one or more connector assemblies 144 are disposed in the header 150. In at least some embodiments, the header 150 defines one or more ports 304 into which a proximal end 306 of the one or more lead bodies 106 with terminals 310 can be inserted, as shown by directional arrows 312, in order to gain access to the connector contacts disposed in the one or more connector assemblies 144.

The one or more connector assemblies 144 each include a connector housing 314 and a plurality of connector contacts 316 disposed therein. Typically, the connector housing 314 defines a port (not shown) that provides access to the plurality of connector contacts 316. In at least some embodiments, one or more of the connector assemblies 144 further includes a retaining element 318 configured and arranged to fasten the corresponding lead body 308 to the connector assembly 144 when the lead body 106 is inserted into the connector assembly 144 to prevent undesired detachment of the lead body 106 from the connector assembly 144. For example, the retaining element 318 may include an aperture through which a fastener (*e.g.,* a set screw, pin, or the like) may be inserted and secured against an inserted lead body or lead extension.

When the one or more lead bodies 106 are inserted into the one or more ports 304, the connector contacts 316 can be aligned with the terminals 310 disposed on the one or more lead bodies 106 to electrically couple the control module 102 to the electrodes (134 of Figure 1) disposed at a distal end of the one or more lead bodies 106. Examples of connector assemblies in control modules are found in, for example, U.S. Patent Nos. 7,244,150 and 8,224,450. In Figure 3C, a lead extension connector assembly 322 is disposed on a lead extension 324. The lead extension connector assembly 322 is shown disposed at a distal end 326 of the lead extension 324. The lead extension connector assembly 322 includes a contact housing 328. The contact housing 328 defines at least one port 330 into which a proximal end 306 of the lead body 106 with terminals 310 can be inserted, as shown by directional arrow 338. The lead extension connector assembly 322 also includes a plurality of connector contacts 340. When the lead body 106 is inserted into the port 330, the connector contacts 340 disposed in the contact housing 328 can be aligned with the terminals 310 on the lead body 106 to electrically couple the lead extension 324 to the electrodes (134 of Figure 1) disposed at a distal end (not shown) of the lead body 106.

The proximal end of a lead extension can be similarly configured and arranged as a proximal end of a lead body. The lead extension 324 may include a plurality of conductive wires (not shown) that electrically couple the connector contacts 340 to a proximal end 348 of the lead extension 324 that is opposite to the distal end 326. The conductive wires disposed in the lead extension 324 can be electrically coupled to a plurality of terminals (not shown) disposed on the proximal end 348 of the lead extension 324. In at least some embodiments, the proximal end 348 of the lead extension 324 is configured and arranged for insertion into a lead extension connector assembly disposed in another lead extension. In other embodiments (as shown in Figure 3C), the proximal end 348 of the lead extension 324 is configured and arranged for insertion into the connector assembly 144 disposed on the control module 102.

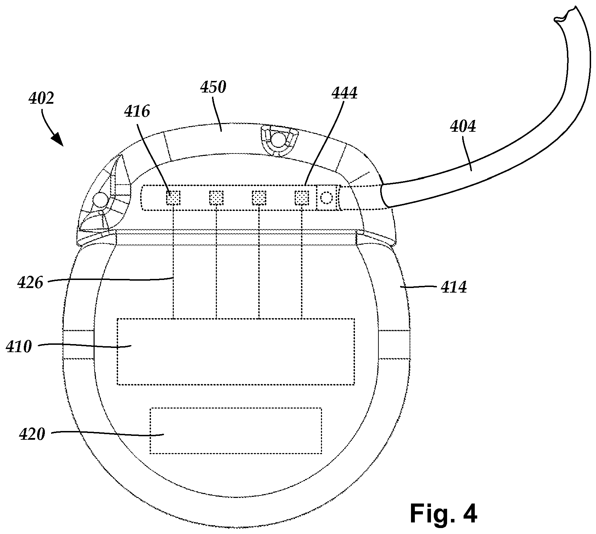

Turning to Figure 4, in at least some embodiments the connector assembly is disposed in a header of the control module. In at least some embodiments, the connector contacts of the connector assembly are electrically coupled to the electronic subassembly within the sealed housing via one or more feedthrough interconnects. Figure 4 is a schematic view of one embodiment of a control module 402 having a header 450 and a sealed housing 414. A connector assembly 444 is disposed in the header 450. An electronic subassembly 410 and an optional power source 420 are disposed in the sealed housing 414.

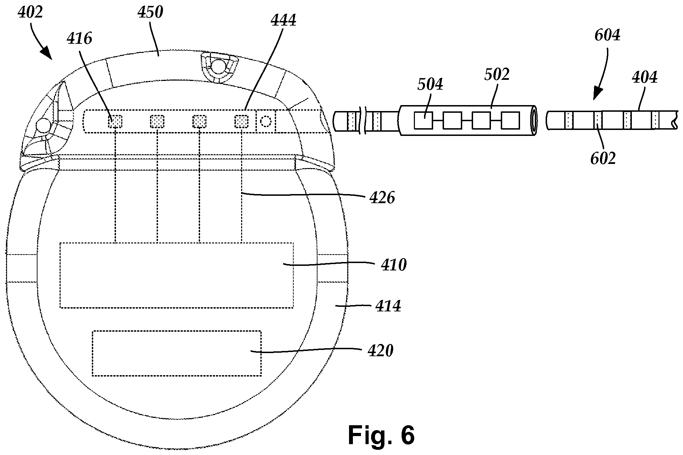

A plurality of connector contacts, such as connector contact 416, are disposed in the connector assembly 444 and are configured and arranged for coupling with terminals (see *e*.*g*., 602 in Figure 6) disposed on a lead 404 when the lead 404 is disposed in the connector assembly 444. In Figure 4, and in other figures, the connector assembly 444 is shown having four connector contacts 416. It will be understood that any suitable number of connector contacts 416 may be utilized including, for example, one, two, three, four, five, six, seven, eight, nine, ten, eleven, twelve, fourteen, sixteen, twenty, twenty-four, thirty-two, or more connector contacts 416. The connector contacts 416 are electrically coupled to the electronic subassembly 410 via a plurality of feedthrough interconnects 426 that extend into the sealed housing 414.

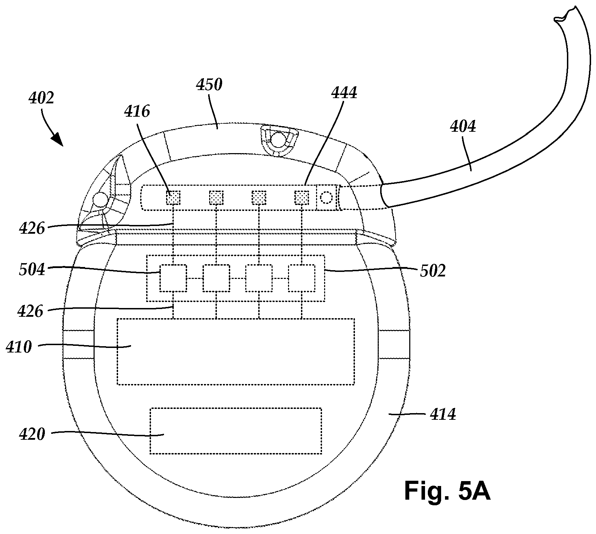

Turning to Figure 5A, conventional electrical stimulation systems may be potentially unsafe for use with magnetic resonance imaging ("MRI") due to the effects of electromagnetic fields (*e.g*., radio frequency fields) in an MRI environment. A common mechanism for causing the electrical interactions between the electrical stimulation system and radiofrequency ("RF") irradiation is common-mode coupling of the applied electromagnetic fields that act as a series of distributed sources along elongated conductive structures, such as leads, or conductors within leads. Common-mode induced RF currents can reach amplitudes of greater than one ampere in MRI environments. Such currents can cause heating and potentially disruptive voltages within electronic circuits, such as electronic circuits disposed within the electronic subassembly.

Some of the effects of RF irradiation may include, for example, inducing current in the lead, causing undesired heating of the lead that may potentially cause tissue damage, undesired or unexpected operation of electronic components, or damage to, or premature failure of, electronic components. Additionally, when an electrical stimulation system is used within an MRI scanner environment, the electrical interactions between the electrical stimulation system and the MRI may cause distortions in images formed by the MRI system.

When the lead is exposed to electromagnetic fields, RF energy may be induced along the length of the lead. When the lead is coupled to the control module, at least some of the RF energy induced along the lead may propagate from the lead to the control module. The RF energy may, for example, propagate between the terminals of the lead and the connector contacts of the connector assembly of the control module. Once the induced RF energy reaches the control module, the RF energy may propagate along a plurality of different conductive paths to the electronic subassembly. Each of the conductive paths may include one or more conductive structures including, for example, a particular connector contact and a particular feedthrough interconnect.

One or more characteristics of the induced RF energy may vary along different conductive structures of the lead (*e.g*., electrodes, lead conductors, and terminals). Variability may be caused by many different factors including, for example, differences in the surroundings of the conductors, differences in presentation to the RF field, or the like). In at least some instances, one or more particular terminals of the plurality of terminals may be more sensitive to RF energy than one or more of the remaining plurality of terminals. In which case, during exposure to RF energy the RF energy that reaches the control module may not be uniformly distributed along each conductive path within the control module. For example, more of the RF energy may propagate along the conductive path of the control module that is electrically coupled to one or more sensitive terminals than the RF energy propagating along one or more of the other, less sensitive terminals.

In some cases, the cumulative amount of RF energy propagating from the lead to the electronic subassembly may be tolerable to the electronic subassembly. However, when the RF energy is more unevenly distributed along a plurality of different conductive paths within the control module, the conductive paths having relatively-high concentrations of RF energy may develop one or more undesirable RF "hot spots" having concentrations of RF energy that are significantly higher than RF energy concentrations along other conductive paths. In some cases, one or more of the "hot spots" may have RF energy concentrations that are high enough to cause damage to electronic circuits within the electronic subassembly. Accordingly, when a plurality of conductive paths electrically couple the lead to the electronic subassembly, it may be desirable to distribute RF energy propagating along the different conductive paths more uniformly to prevent RF "hot spots" from developing.

As herein described, impedance circuitry is disposed between the terminals of the lead and the electronic subassembly of the control module. The impedance circuitry is configured and arranged such that, when the lead is electrically coupled to the control module, the impedance circuitry alters at least one of the characteristics of at least one of multiple conductive paths extending between the lead and the electronic subassembly in order to facilitate a more uniform distribution of undesired RF energy propagating between the lead and the electronic subassembly. Promoting a more even distribution of RF energy may reduce the development of "hot spots" along one or more particular conductive paths. Promoting a more even distribution of RF energy along multiple conductor paths may also increase the cumulative amount of RF energy that can be tolerated by the electronic subassembly during, for example, an MRI procedure.

The impedance circuitry can be disposed in any suitable location between the terminals of the lead and the electronic subassembly. In some embodiments, the impedance circuitry is disposed in the control module. In other embodiments, the impedance circuitry is disposed in an adapter configured and arranged to electrically couple the lead to the control module.

The impedance circuitry provides impedance elements along one or more of the conductive paths. The impedance circuitry may be disposed as elements in series in the conductive paths, or connected between the conductive paths, or connected between conductive paths and one or more common connection points between conductive paths, or any combination thereof. These impedance elements may affect the propagation of RF energy to the electronic subassembly by modifying the propagation amplitude, or the phase (or both) of RF energy passing through the impedance elements if disposed in series in the conductive paths, or modifying the amplitude and phase of the propagating RF energy by shunting a portion of the RF energy between paths, or between paths and a common connection point (or both). In at least some embodiments, impedance elements are connected from each conductive path to a common point consisting of a metal enclosure containing the electronic subassembly.

In at least some embodiments, different impedance elements are provided for each conductive path. In some embodiments, the impedance elements are pre-set such that at least one of the impedance elements provides an impedance along at least one conductive path that is different than an impedance provided along another conductive path by at least one other of the impedance elements. In at least some embodiments, at least one of the impedance elements is adjustable. In which case, the impedance element can be adjusted to promote either increases or decreases of RF energy propagation along individual conductive paths, as needed, to promote a more uniform flow of RF energy along each of the plurality of the conductor paths.

In at least some embodiments, the impedance circuitry is designed to promote modification of the phase of the RF energy propagating along one or more of the conductive paths. The phases can be offset by any suitable number of degrees. It may be advantageous to modify the phases along one or more conductive paths by 180 degrees to provide destructive superposition of at least some of the RF energy input from the lead.

For example, in the case where the lead includes a first terminal and a second terminal, the amplitude of the RF energy propagating along the terminals may be affected by adding a 90 degree phase to the RF energy propagating along the first terminal and reducing the phase by 90 degrees of the RF energy propagating along the second terminal so that the RF energy in the first terminal is offset 180 degrees from the RF energy in the second terminal. In which case, if the RF energy propagating along the first terminal is similar in amplitude to the RF energy propagating along the second terminal, the impedance circuitry may enable the RF energy along the first terminal to cancel out the RF energy along the second terminal so that the net RF energy propagating to the electronic subassembly from the first and second terminals is zero.

It will be understood that a 180-degree phase offset between RF energy propagating along a first conductive structure (*e.g*., a particular terminal) and RF energy propagating along one or more other conductive structures (*e.g*., other terminals) can be achieved using any suitable combination of degrees of phase offsets, where the sums of the offsets between the two or more conductive structures are 180 degrees, or approximately 180 degrees. In the case of two conductive structures promoting phase offsets of propagating RF energy, the relative degrees of phase offsets for the two conductive structures may include, for example, 90-90, 100-80, 110-70, 120-60, 130-50, 140-40, 150-30, 160-20, 170-10, 180-0, or other combination of degrees of phase offset. More generally, the relative degrees of phase offsets may be combined to achieve cancellation of the RF propagating from multiple conductive structures by ensuring that the resulting vector sum of the RF magnitude and phase at the point of combination is zero or near zero.

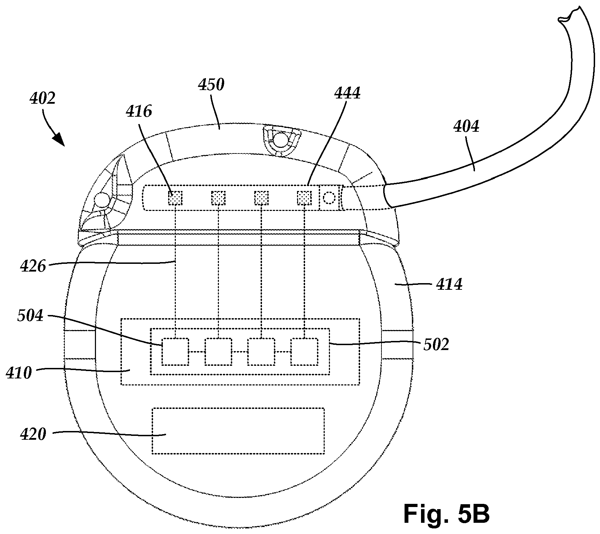

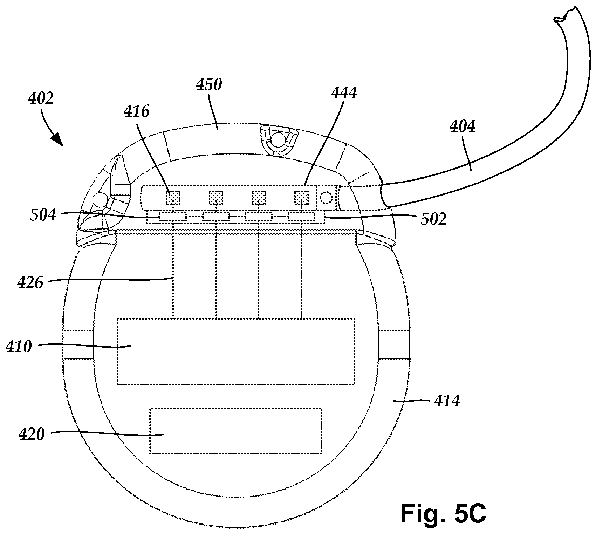

The impedance circuitry can be disposed in any suitable location between the terminals and the electronic subassembly. In at least some embodiments, the impedance circuitry is disposed in the control module (*e.g*., in the header, or in the sealed housing, or both). Figure 5A is a schematic view of one embodiment of impedance circuitry 502 disposed in the control module 402. In Figure 5A, the impedance circuitry 502 is shown disposed in the sealed housing 414 and along the feedthrough interconnects 426, between the connector contacts 416 and the electronic subassembly 410. Figure 5B is a schematic view of another embodiment of the impedance circuitry 502. In Figure 5B, the impedance circuitry 502 is shown disposed on the electronic subassembly 410, between the feedthrough interconnects 426 and the electronics disposed on the electronic subassembly 410. Figure 5C is a schematic view of yet another embodiment of the impedance circuitry 502. In Figure 5C, the impedance circuitry 502 is shown disposed in the header 450, between the connector contacts 416 and the electronic subassembly 410.

In other embodiments, the impedance circuitry is disposed external to the control module. For example, in at least some embodiments the impedance circuitry is formed as a stand-alone device (*e.g.,* an adapter) that can be disposed between the lead and the control module and that electrically couple the terminals of the lead to the connector contacts of the control module via adapter contacts and conductors.

Figure 6 is a schematic view of another embodiment of the impedance circuitry 502. The impedance circuitry 502 is disposed between terminals, such as terminal 602, of the lead 404 and the connector contacts 416. In Figure 6, the impedance circuitry 502 is shown as an adapter that is configured and arranged to receive a proximal end 604 of the lead 404 and that is also configured and arranged for insertion into the connector assembly 444 of the control module 402.

The impedance circuitry 502 (as shown in Figures 5A-6) includes a plurality of impedance elements, such as impedance element 504, electrically coupled (either directly or via one or more other conductive structures) to the feedthrough interconnects 426 and to one another. In at least some embodiments, the number of different impedance elements 504 is equal to the number of conductive paths, in parallel with one another, along which the impedance elements 504 are disposed. For example, in Figures 5A-5C the number of impedance elements 504 is equal to the number of feedthrough interconnects 426. In Figure 6, the number of impedance elements is equal to the number of terminals 602. In Figure 6, the number of impedance elements is also equal to the number of connector contacts 416.

In at least some embodiments, the number of feedthrough interconnects is equal to the number of terminals. In at least some embodiments, the number of feedthrough interconnects is equal to the number of connector contacts. In at least some embodiments, the number of feedthrough interconnects is equal to each of the number of terminals and the number of connector contacts.

In at least some embodiments, the impedance elements 504 are adjustable (*i*.*e*., programmable). Modulation of impedance may enable the impedance circuitry 502 to account for potential changes in RF energy distribution along different conductive structures of a given conductive path due to changes in one or more of: lead properties (*e.g*., multiple lead designs, lead migration over time due to scar tissue formation, soaking, or the like), lead configuration, patient variability, or the like. The impedance modulation can be performed automatically (*e.g*., by sensing change in the power distribution), or manually, or both.

In at least some embodiments, the impedance elements 504 are individually adjusted by impedance matching or mismatching. In which case, each of the impedance elements 504 may have an associated reflection coefficient that either inhibits or promotes RF energy propagation. When, for example, it is desired to increase RF energy propagation along a particular conductive path, a reflection coefficient magnitude of the impedance element 504 disposed on that conductive path may be reduced in magnitude. As an example, the impedance element 504 disposed along a given conductive path in the control module 402 may be designed to have an impedance that matches the impedance of the lead as seen from the connector contact associated with the given conductive path. In which case, RF energy propagating along the lead to the connector contact tends to continue propagating along the given conductive path.

When, for example, it is desired to reduce RF energy propagation along a particular conductive path, the magnitude of the reflection coefficient of the impedance element 504 disposed on that conductive path may be increased (*i.e*., made more positive or more negative, depending on the desired phase). As an example, the impedance element 504 disposed along a given conductor path in the control module 402 may be designed to have an impedance that differs from the impedance of the lead as seen from the connector contact associated with the given conductive path. In which case, at least some of the RF energy propagating along the lead to the connector contact tends to resist propagating along the given conductive path.

Impedance elements 504 may be adjusted using any suitable materials or components. In at least some embodiments, the impedance elements 504 include one or more linear components (*e.g*., resistors, capacitors, inductors, or the like). In at least some embodiments, the impedance elements 504 include, in turn, one or more non-linear elements (*e.g*., diodes, switches, or the like).

In at least some embodiments, one or more of the impedance elements 504 are configured and arranged for promoting modification of the phase of RF energy propagating along the conductive structures on which the one or more impedance elements 504 are disposed. Promotion of phase changes can be achieved using any suitable technique. In at least some embodiments, phase changes are promoted using one or more electrical components (*e.g*., capacitors, inductors, surface acoustic wave filters, or the like). Phase changes may be promoted using other techniques in lieu of, or in addition to, using one or more electrical components. For example, in at least some embodiments, phase changes are promoted using one or more transmission line stubs, or varying the length of a particular conductive structure, or some combination thereof.

In at least some embodiments, the impedance elements 504 are configurable to control RF energy transmission for different lead types which may be used with the control module 402 (*see e.g.,* Figure 1 and Figure 2). In at least some embodiments, the impedance elements 504 may be configured at the time of lead implant (or replacement) to provide improved RF performance with the stimulation system.

In some instances, a given control module may be configured to receive multiple leads or different types of leads. In at least some embodiments, the control module 402 is configured and arranged to receive a parameter set for the impedance elements accompanying each particular lead 402 that is suitable for use with the control module 402. The parameter set may, optionally, configure the impedance elements for a particular interface to that lead for reduced power transmission to tissue, or to the control module 402, or both. These parameter sets may be specific for each physical lead, or for a particular lead model (or type).

It will be understood that the impedance circuitry 504 may be used in conjunction with any suitable implantable electrical medical device that includes a relatively long element with a plurality of conductive paths including, for example, neurostimulators, cardiac stimulators, cochlear implants, or the like.

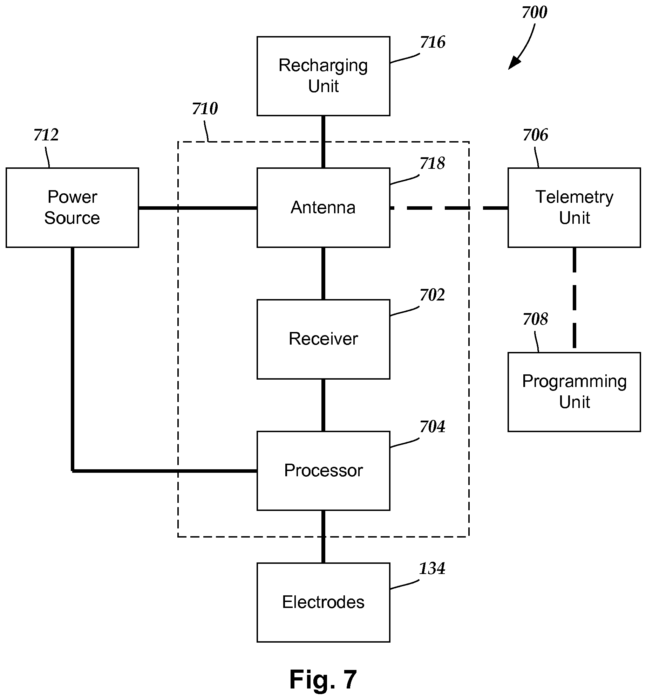

Figure 7 is a schematic overview of one embodiment of components of an electrical stimulation system 700 including an electronic subassembly 710 disposed within a control module. It will be understood that the electrical stimulation system can include more, fewer, or different components and can have a variety of different configurations including those configurations disclosed in the stimulator references cited herein.

Some of the components (for example, power source 712, antenna 718, receiver 702, and processor 704) of the electrical stimulation system can be positioned on one or more circuit boards or similar carriers within a sealed housing of an implantable pulse generator, if desired. Any power source 712 can be used including, for example, a battery such as a primary battery or a rechargeable battery. Examples of other power sources include super capacitors, nuclear or atomic batteries, mechanical resonators, infrared collectors, thermally-powered energy sources, flexural powered energy sources, bioenergy power sources, fuel cells, bioelectric cells, osmotic pressure pumps, and the like including the power sources described in U.S. Patent No. 7,437,193. As another alternative, power can be supplied by an external power source through inductive coupling via the optional antenna 718 or a secondary antenna. The external power source can be in a device that is mounted on the skin of the user or in a unit that is provided near the user on a permanent or periodic basis.

If the power source 712 is a rechargeable battery, the battery may be recharged using the optional antenna 718, if desired. Power can be provided to the battery for recharging by inductively coupling the battery through the antenna to a recharging unit 716 external to the user. Examples of such arrangements can be found in the references identified above.

In one embodiment, electrical current is emitted by the electrodes 134 on the paddle or lead body to stimulate nerve fibers, muscle fibers, or other body tissues near the electrical stimulation system. A processor 704 is generally included to control the timing and electrical characteristics of the electrical stimulation system. For example, the processor 704 can, if desired, control one or more of the timing, frequency, strength, duration, and waveform of the pulses. In addition, the processor 704 can select which electrodes can be used to provide stimulation, if desired. In some embodiments, the processor 704 may select which electrode(s) are cathodes and which electrode(s) are anodes. In some embodiments, the processor 704 may be used to identify which electrodes provide the most useful stimulation of the desired tissue.

Any processor can be used and can be as simple as an electronic device that, for example, produces pulses at a regular interval or the processor can be capable of receiving and interpreting instructions from an external programming unit 708 that, for example, allows modification of pulse characteristics. In the illustrated embodiment, the processor 704 is coupled to a receiver 702 which, in turn, is coupled to the optional antenna 718. This allows the processor 704 to receive instructions from an external source to, for example, direct the pulse characteristics and the selection of electrodes, if desired.

In one embodiment, the antenna 718 is capable of receiving signals (*e.g*., RF signals) from an external telemetry unit 706 which is programmed by a programming unit 708. The programming unit 708 can be external to, or part of, the telemetry unit 706. The telemetry unit 706 can be a device that is worn on the skin of the user or can be carried by the user and can have a form similar to a pager, cellular phone, or remote control, if desired. As another alternative, the telemetry unit 706 may not be worn or carried by the user but may only be available at a home station or at a clinician's office. The programming unit 708 can be any unit that can provide information to the telemetry unit 706 for transmission to the electrical stimulation system 700. The programming unit 708 can be part of the telemetry unit 706 or can provide signals or information to the telemetry unit 706 via a wireless or wired connection. One example of a suitable programming unit is a computer operated by the user or clinician to send signals to the telemetry unit 706.

The signals sent to the processor 704 via the antenna 718 and receiver 702 can be used to modify or otherwise direct the operation of the electrical stimulation system. For example, the signals may be used to modify the pulses of the electrical stimulation system such as modifying one or more of pulse duration, pulse frequency, pulse waveform, and pulse strength. The signals may also direct the electrical stimulation system 700 to cease operation, to start operation, to start charging the battery, or to stop charging the battery. In other embodiments, the stimulation system does not include an antenna 718 or receiver 702 and the processor 704 operates as programmed.

Optionally, the electrical stimulation system 700 may include a transmitter (not shown) coupled to the processor 704 and the antenna 718 for transmitting signals back to the telemetry unit 706 or another unit capable of receiving the signals. For example, the electrical stimulation system 700 may transmit signals indicating whether the electrical stimulation system 700 is operating properly or not or indicating when the battery needs to be charged or the level of charge remaining in the battery. The processor 704 may also be capable of transmitting information about the pulse characteristics so that a user or clinician can determine or verify the characteristics.

The invention is set out in the appended claims that follow:

## Claims

1. An implantable medical device system (100) for providing electrical stimulation, the system comprising:
a control module (102) configured and arranged to electrically couple to a lead, (106) the control
module comprising
a sealed housing (414) having an interior and an exterior, an electronic subassembly (410) disposed in the interior of the housing,
a connector assembly (444) coupled to the exterior of the housing, the connector assembly defining a port configured and arranged for receiving the lead,
a plurality of connector contacts (416) disposed in the port, the connector contacts configured and arranged to electrically couple with terminals of the lead when the lead is operationally received by the port, wherein the plurality of connector contacts comprise a first connector contact and a second connector contact, and
a plurality of feedthrough interconnects (426) extending from the connector assembly into the interior of the sealed housing, the plurality of feedthrough interconnects electrically coupling the plurality of connector contacts to the electronic subassembly, wherein the plurality of feedthrough interconnects comprises a first feedthrough interconnect and a second feedthrough interconnect, the first feedthrough interconnect electrically coupling the first connector contact to the electronic subassembly and the second feedthrough interconnect electrically coupling the second connector contact to the electronic subassembly; and
impedance circuitry (502) disposed in the control module and configured and arranged for modulating impedance associated with terminals and conductors of the lead, the impedance circuitry comprising a plurality of impedance elements, wherein each of the plurality of impedance elements is electrically coupled to a different feedthrough interconnect of the plurality of feedthrough interconnects, the plurality of impedance elements each having pre-defined impedances, the plurality of impedance elements comprising a first impedance element electrically coupled to the first feedthrough interconnect and a second impedance element electrically coupled to the second feedthrough interconnect, wherein the pre-defined impedance of the first impedance element is different than the pre-defined impedance of the second impedance element, wherein the control module defines a plurality of conductive paths extending between the lead and electronic subassembly, each conductive path comprising a connector contact and a feedthrough interconnect, wherein the impedance circuitry is configured and arranged to alter at least one of the characteristics of at least one of the plurality of conductive paths in order to facilitate a more uniform distribution of undesired radiofrequency energy propagating between the lead and the electronic subassembly when the implantable medical device system is exposed to applied electromagnetic fields.

2. The system of claim 1, wherein the impedance circuitry is disposed in the connector assembly.

3. The system of claim 1, wherein the impedance circuitry is disposed in the interior of the sealed housing.

4. The system of claim 1, wherein the impedance circuitry is disposed on the electronic subassembly.

5. The system of claim 1, wherein the plurality of impedance elements each comprise at least one of a resistor, a capacitor, or an inductor.

6. The system of claim 1, wherein the plurality of impedance elements each comprise at least one of a diode or a switch.

7. The system of claim 1, further comprising a lead configured and arranged for insertion into a patient, the lead comprising
a lead body having a distal end, a proximal end, and a longitudinal length,
a plurality of electrodes disposed on the distal end of the lead body,
a plurality of terminals disposed on the proximal end of the lead body, the plurality of terminals comprising a first terminal and a second terminal, wherein the first terminal is configured and arranged for electrically coupling with the first connector contact when the lead is operationally received by the port, and wherein the second terminal is configured and arranged for electrically coupling with the second connector contact when the lead is operationally received by the port, and
a plurality of conductors electrically coupling the plurality of electrodes to at least one of the terminals.

8. The system of claim 7, wherein the first impedance element is configured and arranged to promote modification of a phase of radiofrequency energy propagating along the first feedthrough interconnect when the lead is exposed to an applied electromagnetic field and resulting radiofrequency energy is propagated from the lead to the first feedthrough interconnect.

9. The system of claim 7, wherein the first impedance element is configured and arranged to promote modification of a phase of radiofrequency energy propagating along the first feedthrough interconnect with respect to a phase of radiofrequency energy propagating along the second feedthrough interconnect to provide destructive superposition between the radiofrequency energy propagating along the first feedthrough interconnect and the RF energy propagating along the second feedthrough interconnect when the lead is exposed to an applied electromagnetic field and resulting radiofrequency energy is propagated from the lead to each of the first feedthrough interconnect and to the second feedthrough interconnect.

10. The system of claim 7, wherein the first impedance element comprises at least one of a capacitor or an inductor for promoting modification of the phase of the radiofrequency energy propagating along the first feedthrough interconnect when the lead is exposed to applied electromagnetic fields resulting in radiofrequency energy propagating along the first feedthrough interconnect.

11. The system of claim 7, wherein the first impedance element comprises at least one transmission line stub for promoting modification of a phase of radiofrequency energy propagating along the first feedthrough interconnect when the lead is exposed to an applied electromagnetic field resulting in radiofrequency energy propagating along the first feedthrough interconnect.

12. The system of claim 7, wherein the first impedance element has a first conductive length and the second impedance element has a second conductive length that is different from the first conductive length, the difference in conductive length between the first impedance element and the second impedance element providing destructive superposition between radiofrequency energy propagating along the first feedthrough interconnect and radiofrequency energy propagating along the second feedthrough interconnect when the lead is exposed to an applied electromagnetic field resulting in radiofrequency energy propagating along the first feedthrough interconnect.

13. An implantable medical device system (100) for providing electrical stimulation, the system comprising:
a control module (102) configured and arranged to electrically couple to a lead (106), the control module comprising
a sealed housing (414) having an interior and an exterior, an electronic subassembly (410) disposed in the interior of the housing,
a connector assembly (444) coupled to the exterior of the housing, the connector assembly defining a port configured and arranged for receiving the lead,
a plurality of connector contacts (416) disposed in the port, the connector contacts configured and arranged to electrically couple with terminals of the lead when the lead is operationally received by the port, and
a plurality of feedthrough interconnects (426) extending from the connector assembly into the interior of the sealed housing, the plurality of feedthrough interconnects electrically coupling the plurality of connector contacts to the electronic subassembly;
and
impedance circuitry (502) disposed in the control module and configured and arranged for modulating impedance associated with terminals and conductors of the lead, the impedance circuitry comprising a plurality of impedance elements, wherein each of the plurality of impedance elements is electrically coupled to a different feedthrough interconnect of the plurality of feedthrough interconnects, and wherein at least one of the impedance elements is configured and arranged for enabling adjustment of impedance along the feedthrough interconnect to which the at least one of the impedance elements is electrically coupled, wherein the control module defines a plurality of conductive paths extending between the lead and electronic subassembly, each conductive path comprising a connector contact and a feedthrough interconnect, wherein the impedance circuitry is configured and arranged to alter at least one of the characteristics of at least one of the plurality of conductive paths in order to facilitate a more uniform distribution of undesired radiofrequency energy propagating between the lead and the electronic subassembly when the implantable medical device system is exposed to applied electromagnetic fields.

14. The system of claim 13, wherein at least one of the impedance elements is configured and arranged to promote modification of a phase of radiofrequency energy propagating along the at least one feedthrough interconnect to which the at least one impedance element is electrically coupled when radiofrequency energy is propagated to the control module from the electrically-coupled lead.

## Patentansprüche

1. Implantierbares Medizinproduktsystem (100) zur Bereitstellung von elektrischer Stimulation, wobei das System aufweist:
ein Steuermodul (102), das konfiguriert und angeordnet ist, um mit einer Leitung (106) elektrisch gekoppelt zu werden, wobei das Steuermodul aufweist:
ein abgedichtetes Gehäuse (414) mit einem Inneren und einem Äußeren,
eine elektronische Teilanordnung (410), die im Inneren des Gehäuses angeordnet ist,
eine Verbinderanordnung (444), die mit dem Äußeren des Gehäuses gekoppelt ist, wobei die Verbinderanordnung einen Port definiert, der zum Aufnehmen der Leitung konfiguriert und angeordnet ist,
mehrere Verbinderkontakte (416), die im Port angeordnet sind, wobei die Verbinderkontakte so konfiguriert und angeordnet sind, dass sie mit Anschlüssen der Leitung elektrisch gekoppelt sind, wenn die Leitung durch den Port betriebsbereit aufgenommen ist, wobei die mehreren Verbinderkontakte einen ersten Verbinderkontakt und einen zweiten Verbinderkontakt aufweisen, und
mehrere Durchführungszwischenverbindungen (426), die sich von der Verbinderanordnung in das Innere des abgedichteten Gehäuses erstrecken, wobei die mehreren Durchführungszwischenverbindungen die mehreren Verbinderkontakte mit der elektronischen Teilanordnung elektrisch koppeln, wobei die mehreren Durchführungszwischenverbindungen eine erste Durchführungszwischenverbindung und eine zweite Durchführungszwischenverbindung aufweisen, wobei die erste Durchführungszwischenverbindung den ersten Verbinderkontakt mit der elektronischen Teilanordnung elektrisch koppelt und die zweite Durchführungszwischenverbindung den zweiten Verbinderkontakt mit der elektronischen Teilanordnung elektrisch koppelt; und
einen Impedanzschaltungsaufbau (502), der im Steuermodul angeordnet und zum Modulieren von Impedanz, die Anschlüssen und Leitern der Leitung zugehörig ist, konfiguriert und angeordnet ist, wobei der Impedanzschaltungsaufbau mehrere Impedanzelemente aufweist, wobei jedes der mehreren Impedanzelemente mit einer unterschiedlichen Durchführungszwischenverbindung der mehreren Durchführungszwischenverbindungen elektrisch gekoppelt ist, wobei die mehreren Impedanzelemente jeweils vorab definierte Impedanzen haben, die mehreren Impedanzelemente ein erstes Impedanzelement, das mit der ersten Durchführungszwischenverbindung elektrisch gekoppelt ist, und ein zweites Impedanzelement, das mit der zweiten Durchführungszwischenverbindung elektrisch gekoppelt ist, aufweisen, wobei sich die vorab definierte Impedanz des ersten Impedanzelements von der vorab definierten Impedanz des zweiten Impedanzelements unterscheidet, wobei das Steuermodul mehrere Leitwege definiert, die sich zwischen der Leitung und der elektronischen Teilanordnung erstrecken, wobei jeder Leitweg einen Verbinderkontakt und eine Durchführungszwischenverbindung aufweist, wobei der Impedanzschaltungsaufbau so konfiguriert und angeordnet ist, dass er mindestens einen der Kennwerte mindestens eines der Leitwege abändert, um eine gleichmäßigere Verteilung unerwünschter Hochfrequenzenergie zu erleichtern, die sich zwischen der Leitung und der elektronischen Teilanordnung ausbreitet, wenn das implantierbare Medizinproduktsystem angelegten elektromagnetischen Feldern ausgesetzt ist.

2. System nach Anspruch 1, wobei der Impedanzschaltungsaufbau in der Verbinderanordnung angeordnet ist.

3. System nach Anspruch 1, wobei der Impedanzschaltungsaufbau im Inneren des abgedichteten Gehäuses angeordnet ist.

4. System nach Anspruch 1, wobei der Impedanzschaltungsaufbau auf der elektronischen Teilanordnung angeordnet ist.

5. System nach Anspruch 1, wobei die mehreren Impedanzelemente jeweils einen Widerstand, einen Kondensator und/oder einen Induktor aufweisen.

6. System nach Anspruch 1, wobei die mehreren Impedanzelemente jeweils eine Diode und/oder einen Schalter aufweisen.

7. System nach Anspruch 1, das ferner eine Leitung aufweist, die zur Einführung in einen Patienten konfiguriert und angeordnet ist, wobei die Leitung aufweist:
einen Leitungskörper mit einem distalen Ende, einem proximalen Ende und einer Längslänge,
mehrere Elektroden, die auf dem distalen Ende des Leitungskörpers angeordnet sind,
mehrere Anschlüsse, die auf dem proximalen Ende des Leitungskörpers angeordnet sind, wobei die mehreren Anschlüsse einen ersten Anschluss und einen zweiten Anschluss aufweisen, wobei der erste Anschluss zum elektrischen Koppeln mit dem ersten Verbinderkontakt konfiguriert und angeordnet ist, wenn die Leitung durch den Port betriebsbereit aufgenommen ist, und wobei der zweite Anschluss zum elektrischen Koppeln mit dem zweiten Verbinderkontakt konfiguriert und angeordnet ist, wenn die Leitung durch den Port betriebsbereit aufgenommen ist, und
mehrere Leiter, die die mehreren Elektroden mit mindestens einem der Anschlüsse elektrisch koppeln.

8. System nach Anspruch 7, wobei das erste Impedanzelement so konfiguriert und angeordnet ist, dass es Modifizierung einer Phase von Hochfrequenzenergie fördert, die sich entlang der ersten Durchführungszwischenverbindung ausbreitet, wenn die Leitung einem angelegten elektromagnetischen Feld ausgesetzt ist und sich resultierende Hochfrequenzenergie von der Leitung zur ersten Durchführungszwischenverbindung ausbreitet.

9. System nach Anspruch 7, wobei das erste Impedanzelement so konfiguriert und angeordnet ist, dass es Modifizierung einer Phase von Hochfrequenzenergie, die sich entlang der ersten Durchführungszwischenverbindung ausbreitet, im Hinblick auf eine Phase von Hochfrequenzenergie fördert, die sich entlang der zweiten Durchführungszwischenverbindung ausbreitet, um für auslöschende Überlagerung zwischen der Hochfrequenzenergie, die sich entlang der ersten Durchführungszwischenverbindung ausbreitet, und der Hochfrequenzenergie zu sorgen, die sich entlang der zweiten Durchführungszwischenverbindung ausbreitet, wenn die Leitung einem angelegten elektromagnetischen Feld ausgesetzt ist und sich resultierende Hochfrequenzenergie von der Leitung jeweils zur ersten Durchführungszwischenverbindung und zur zweiten Durchführungszwischenverbindung ausbreitet.

10. System nach Anspruch 7, wobei das erste Impedanzelement einen Kondensator und/oder einen Induktor zum Fördern von Modifizierung der Phase der Hochfrequenzenergie aufweist, die sich entlang der ersten Durchführungszwischenverbindung ausbreitet, wenn die Leitung angelegten elektromagnetischen Feldern ausgesetzt ist, die dazu führen, dass sich Hochfrequenzenergie entlang der ersten Durchführungszwischenverbindung ausbreitet.

11. System nach Anspruch 7, wobei das erste Impedanzelement mindestens einen Übertragungsleitungsabzweig zum Fördern von Modifizierung einer Phase von Hochfrequenzenergie aufweist, die sich entlang der ersten Durchführungszwischenverbindung ausbreitet, wenn die Leitung einem angelegten elektromagnetischen Feld ausgesetzt ist, was dazu führt, dass sich Hochfrequenzenergie entlang der ersten Durchführungszwischenverbindung ausbreitet.

12. System nach Anspruch 7, wobei das erste Impedanzelement eine erste Leitlänge hat und das zweite Impedanzelement eine zweite Leitlänge hat, die sich von der ersten Leitlänge unterscheidet, wobei die Leitlängendifferenz zwischen dem ersten Impedanzelement und dem zweiten Impedanzelement für auslöschende Überlagerung zwischen Hochfrequenzenergie, die sich entlang der ersten Durchführungszwischenverbindung ausbreitet, und Hochfrequenzenergie sorgt, die sich entlang der zweiten Durchführungszwischenverbindung ausbreitet, wenn die Leitung einem angelegten elektromagnetischen Feld ausgesetzt ist, was dazu führt, dass sich Hochfrequenzenergie entlang der ersten Durchführungszwischenverbindung ausbreitet.

13. Implantierbares Medizinproduktsystem (100) zur Bereitstellung von elektrischer Stimulation, wobei das System aufweist:
ein Steuermodul (102), das konfiguriert und angeordnet ist, um mit einer Leitung (106) elektrisch gekoppelt zu werden, wobei das Steuermodul aufweist:
ein abgedichtetes Gehäuse (414) mit einem Inneren und einem Äußeren,
eine elektronische Teilanordnung (410), die im Inneren des Gehäuses angeordnet ist,
eine Verbinderanordnung (444), die mit dem Äußeren des Gehäuses gekoppelt ist, wobei die Verbinderanordnung einen Port definiert, der zum Aufnehmen der Leitung konfiguriert und angeordnet ist,
mehrere Verbinderkontakte (416), die im Port angeordnet sind, wobei die Verbinderkontakte so konfiguriert und angeordnet sind, dass sie mit Anschlüssen der Leitung elektrisch gekoppelt sind, wenn die Leitung durch den Port betriebsbereit aufgenommen ist, und
mehrere Durchführungszwischenverbindungen (426), die sich von der Verbinderanordnung in das Innere des abgedichteten Gehäuses erstrecken, wobei die mehreren Durchführungszwischenverbindungen die mehreren Verbinderkontakte mit der elektronischen Teilanordnung elektrisch koppeln; und
einen Impedanzschaltungsaufbau (502), der im Steuermodul angeordnet und zum Modulieren von Impedanz, die Anschlüssen und Leitern der Leitung zugeordnet ist, konfiguriert und angeordnet ist, wobei der Impedanzschaltungsaufbau mehrere Impedanzelemente aufweist, wobei jedes der mehreren Impedanzelemente mit einer unterschiedlichen Durchführungszwischenverbindung der mehreren Durchführungszwischenverbindungen elektrisch gekoppelt ist, und wobei mindestens eines der Impedanzelemente zum Ermöglichen von Impedanzeinstellung entlang der Durchführungszwischenverbindung konfiguriert und angeordnet ist, mit der das mindestens eine der Impedanzelemente elektrisch gekoppelt ist, wobei das Steuermodul mehrere Leitwege definiert, die sich zwischen der Leitung und elektronischen Teilanordnung erstrecken, wobei jeder Leitweg einen Verbinderkontakt und eine Durchführungszwischenverbindung aufweist, wobei der Impedanzschaltungsaufbau so konfiguriert und angeordnet ist, dass er mindestens einen der Kennwerte mindestens eines der Leitwege abändert, um eine gleichmäßigere Verteilung unerwünschter Hochfrequenzenergie zu erleichtern, die sich zwischen der Leitung und der elektronischen Teilanordnung ausbreitet, wenn das implantierbare Medizinproduktsystem angelegten elektromagnetischen Feldern ausgesetzt ist.

14. System nach Anspruch 13, wobei mindestens eines der Impedanzelemente so konfiguriert und angeordnet ist, dass es Modifizierung einer Phase von Hochfrequenzenergie fördert, die sich entlang der mindestens einen Durchführungszwischenverbindung ausbreitet, mit der das mindestens eine Impedanzelement elektrisch gekoppelt ist, wenn sich Hochfrequenzenergie von der elektrisch gekoppelten Leitung zum Steuermodul ausbreitet.

## Revendications

1. Système de dispositif médical implantable (100) pour fournir une stimulation électrique, le système comprenant :
un module de commande (102) configuré et agencé pour un couplage électrique à un câble (106), le module de commande comprenant
un logement fermé hermétiquement (414) comportant un intérieur et un extérieur,
un sous-ensemble électronique (410) disposé à l'intérieur du logement,
un ensemble de connecteur (444) couplé à l'extérieur du logement, l'ensemble de connecteur définissant un orifice configuré et agencé pour recevoir le câble,
une pluralité de contacts de connecteur (416) disposés dans l'orifice, les contacts de connecteur étant configurés et agencés pour un couplage électrique aux bornes du câble lorsque le câble est reçu de manière fonctionnelle par l'orifice, dans lequel la pluralité de contacts de connecteur comprend un premier contact de connecteur et un deuxième contact de connecteur, et
une pluralité d'interconnexions traversantes (426) s'étendant de l'ensemble de connecteur à l'intérieur du logement fermé hermétiquement, la pluralité d'interconnexions traversantes couplant électriquement la pluralité de contacts de connecteur au sous-ensemble électronique, dans lequel la pluralité d'interconnexions traversantes comprend une première interconnexion traversante et une deuxième interconnexion traversante, la première interconnexion traversante couplant électriquement le premier contact de connecteur au sous-ensemble électronique et la deuxième interconnexion traversante couplant électriquement le deuxième contact de connecteur au sous-ensemble électronique ; et
des éléments de circuit d'impédance (502) disposés dans le module de commande et configurés et agencés pour moduler l'impédance associée aux bornes et aux conducteurs du câble, les éléments de circuit d'impédance comprenant une pluralité d'éléments d'impédance, dans lequel chacun de la pluralité d'éléments d'impédance est couplé électriquement à une interconnexion traversante différente de la pluralité d'interconnexions traversantes, la pluralité d'éléments d'impédance ayant chacun des impédances prédéfinies, la pluralité d'éléments d'impédance comprenant un premier élément d'impédance couplé électriquement à la première interconnexion traversante et un deuxième élément d'impédance couplé électriquement à la deuxième interconnexion traversante, dans lequel l'impédance prédéfinie du premier élément d'impédance est différente de l'impédance prédéfinie du deuxième élément d'impédance, dans lequel le module de commande définit une pluralité de trajets conducteurs s'étendant entre le câble et un sous-ensemble électronique, chaque trajet conducteur comprenant un contact de connecteur et une interconnexion traversante, dans lequel les éléments de circuit d'impédance sont configurés et agencés pour modifier au moins l'une des caractéristiques d'au moins l'un de la pluralité de trajets conducteurs afin de faciliter une distribution plus uniforme de l'énergie radiofréquence indésirable se propageant entre le câble et le sous-ensemble électronique lorsque le système de dispositif médical implantable est exposé à des champs électromagnétiques appliqués.

2. Système selon la revendication 1, dans lequel les éléments de circuit d'impédance sont disposés dans l'ensemble de connecteur.

3. Système selon la revendication 1, dans lequel les éléments de circuit d'impédance sont disposés à l'intérieur du logement fermé hermétiquement.

4. Système selon la revendication 1, dans lequel les éléments de circuit d'impédance sont disposés sur le sous-ensemble électronique.

5. Système selon la revendication 1, dans lequel la pluralité d'éléments d'impédance comprennent chacun au moins l'un d'une résistance, d'un condensateur, ou d'une inductance.

6. Système selon la revendication 1, dans lequel la pluralité d'éléments d'impédance comprennent chacun au moins l'un d'une diode ou d'un commutateur.

7. Système selon la revendication 1, comprenant en outre un câble configuré et agencé pour une insertion dans un patient, le câble comprenant
un corps de câble ayant une extrémité distale, une extrémité proximale, et une longueur longitudinale,
une pluralité d'électrodes disposées sur l'extrémité distale du corps de câble,
une pluralité de bornes disposées sur l'extrémité proximale du corps de câble, la pluralité de bornes comprenant une première borne et une deuxième borne, dans lequel la première borne est configurée et agencée pour un couplage électrique au premier contact de connecteur lorsque le câble est reçu de manière fonctionnelle par l'orifice, et dans lequel la deuxième borne est configurée et agencée pour un couplage électrique au deuxième contact de connecteur lorsque le câble est reçu de manière fonctionnelle par l'orifice, et
une pluralité de conducteurs couplant électriquement la pluralité d'électrodes à au moins l'une des bornes.

8. Système selon la revendication 7, dans lequel le premier élément d'impédance est configuré et agencé pour favoriser la modification d'une phase de l'énergie radiofréquence se propageant le long de la première interconnexion traversante lorsque le câble est exposé à un champ électromagnétique appliqué et que l'énergie radiofréquence résultante se propage du câble vers la première interconnexion traversante.

9. Système selon la revendication 7, dans lequel le premier élément d'impédance est configuré et agencé pour favoriser la modification d'une phase de l'énergie radiofréquence se propageant le long de la première interconnexion traversante par rapport à une phase de l'énergie radiofréquence se propageant le long de la deuxième interconnexion traversante pour obtenir une superposition destructive entre l'énergie radiofréquence se propageant le long de la première interconnexion traversante et l'énergie RF se propageant le long de la deuxième interconnexion traversante lorsque le câble est exposé à un champ électromagnétique appliqué et que l'énergie radiofréquence résultante se propage du câble vers chacune de la première interconnexion traversante et de la deuxième interconnexion traversante.

10. Système selon la revendication 7, dans lequel le premier élément d'impédance comprend au moins l'un d'un condensateur ou d'une inductance pour favoriser la modification de la phase de l'énergie radiofréquence se propageant le long de la première interconnexion traversante lorsque le câble est exposé à des champs électromagnétiques appliqués résultant en la propagation de l'énergie radiofréquence le long de la première interconnexion traversante.

11. Système selon la revendication 7, dans lequel le premier élément d'impédance comprend au moins un tronçon de ligne de transmission pour favoriser la modification d'une phase de l'énergie radiofréquence se propageant le long de la première interconnexion traversante lorsque le câble est exposé à un champ électromagnétique appliqué résultant en la propagation de l'énergie radiofréquence le long de la première interconnexion traversante.

12. Système selon la revendication 7, dans lequel le premier élément d'impédance a une première longueur de conduction et le deuxième élément d'impédance a une deuxième longueur de conduction qui est différente de la première longueur de conduction, la différence de longueur de conduction entre le premier élément d'impédance et le deuxième élément d'impédance permettant une superposition destructive entre l'énergie radiofréquence se propageant le long de la première interconnexion traversante et l'énergie radiofréquence se propageant le long de la deuxième interconnexion traversante lorsque le câble est exposé à un champ électromagnétique appliqué résultant en la propagation de l'énergie radiofréquence le long de la première interconnexion traversante.

13. Système de dispositif médical implantable (100) pour fournir une stimulation électrique, le système comprenant :
un module de commande (102) configuré et agencé pour un couplage électrique à un câble (106), le module de commande comprenant
un logement fermé hermétiquement (414) ayant un intérieur et un extérieur,
un sous-ensemble électronique (410) disposé à l'intérieur du logement,
un ensemble de connecteur (444) couplé à l'extérieur du logement, l'ensemble de connecteur définissant un orifice configuré et agencé pour recevoir le câble,
une pluralité de contacts de connecteur (416) disposés dans l'orifice, les contacts de connecteur étant configurés et agencés pour un couplage électrique aux bornes du câble lorsque le câble est reçu de manière fonctionnelle par l'orifice, et
une pluralité d'interconnexions traversantes (426) s'étendant de l'ensemble de connecteur à l'intérieur du logement fermé hermétiquement, la pluralité d'interconnexions traversantes couplant électriquement la pluralité de contacts de connecteur au sous-ensemble électronique ; et
des éléments de circuit d'impédance (502) disposés dans le module de commande et configurés et agencés pour moduler l'impédance associée aux bornes et aux conducteurs du câble, les éléments de circuit d'impédance comprenant une pluralité d'éléments d'impédance, dans lequel chacun de la pluralité d'éléments d'impédance est couplé électriquement à une interconnexion traversante différente de la pluralité d'interconnexions traversantes, et dans lequel au moins l'un des éléments d'impédance est configuré et agencé pour permettre l'ajustement de l'impédance le long de l'interconnexion traversante à laquelle ledit au moins un des éléments d'impédance est couplé électriquement, dans lequel le module de commande définit une pluralité de trajets conducteurs s'étendant entre le câble et le sous-ensemble électronique, chaque trajet conducteur comprenant un contact de connecteur et une interconnexion traversante, dans lequel les éléments de circuit d'impédance sont configurés et agencés pour modifier au moins l'une des caractéristiques d'au moins l'un de la pluralité de trajets conducteurs afin de faciliter une répartition plus uniforme de l'énergie radiofréquence indésirable se propageant entre le câble et le sous-ensemble électronique lorsque le système de dispositif médical implantable est exposé à des champs électromagnétiques appliqués.

14. Système selon la revendication 13, dans lequel au moins l'un des éléments d'impédance est configuré et agencé pour favoriser la modification d'une phase de l'énergie radiofréquence se propageant le long de ladite au moins une interconnexion traversante à laquelle ledit au moins un élément d'impédance est couplé électriquement lorsque l'énergie radiofréquence se propage vers le module de commande à partir du câble couplé électriquement.
